# EUROPEAN PATENT APPLICATION

(11) **EP 2 554 137 A2**
(43) Date of publication of application: **06.02.2013**
(21) Application number: 12178673.5
(22) Date of filing: 31.07.2012
(51) Int. Cl.: A61B 19/00

(54) **Method and apparatus for processing medical image, and robotic surgery system using image guidance**

(30) Priority: 02.08.2011 KR 20110076993
(71) Applicant: Samsung Electronics Co., Ltd., Suwon-si, Gyeonggi-do, 443-742 (KR)
(72) Inventor: Park, Dong-Ryeol, Gyeonggi-do (KR); Kim, Yeon-Ho, Gyeonggi-do (KR)
(74) Representative: Hylarides, Paul Jacques

(57) **Abstract**

A synthesis image in which medical images captured using different medical image capturing apparatuses are registered is generated by mapping the medical images to each other. The synthesis image may be used for image guidance while a diagnosis or a robotic surgery of a patient is being performed.

## Description

### BACKGROUND

### 1. Field

One or more embodiments of the present disclosure relate to a method and apparatus for processing a medical image, and a robotic surgery system using image guidance.

### 2. Description of the Related Art

Unlike manually performed abdominal operations, robotic surgeries allow surgeons to view a part to be operated on while inside the body of a patient through a screen display of a monitor instead of having to directly view the part to be operated on with the naked eye. While surgeons perform robotic surgeries after having perceived a part to be operated on using computed tomography (CT) images, magnetic resonance imaging (MRI) images, and ultrasound images, there is a limitation in that the robotic surgeries significantly depend on the experience of the surgeons. That is, because the CT images, MRI images, and ultrasound images are taken prior to the surgery, the surgery depends on the experience of the surgeon. In addition, robotic surgery has been performed while viewing actual images in the body of a patient by inserting a laparoscope into the body to acquire images of a part to be operated on. However, because images of a part to be operated on that are acquired with an endoscope, such as a laparoscope, are only images of surfaces of a patient's internal organs, when the part to be operated is not viewed by the scope because it is hidden by other organs or because it is inside other organs, it is difficult for a surgeon to perceive a correct position and shape of the part to be operated on.

### SUMMARY

One or more embodiments of the present disclosure relate to a method and apparatus for processing a medical image, a computer-readable recording medium storing a computer-readable program for executing the method in a computer or processor, and a robotic surgery system using image guidance based on the processed medical image.

Additional aspects will be set forth in part in the description which follows and, in part, will be apparent from the description, or may be learned by practice of the presented embodiments.

According to one or more embodiments, a method of processing a medical image includes: acquiring medical images captured using a plurality of multi-modal medical image capturing apparatuses with respect to a predetermined organ; extracting surface information of the predetermined organ, which is contained in each of the medical images, from each of the medical images; mapping the medical images by using the extracted surface information; and generating a synthesis image in which the medical images have been registered, based on the mapping result and outputting or displaying the synthesis image.

According to another embodiment, there is provided a computer-readable recording medium storing a computer-readable program for executing the medical image processing method in a computer.

According to one or more other embodiments, an apparatus for processing a medical image includes: an image acquisition unit for acquiring medical images captured using a plurality of multi-modal medical image capturing apparatuses with respect to a predetermined organ; a surface information extractor for extracting surface information of the predetermined organ, which is contained in each of the medical images, from each of the medical images; an image mapping unit for mapping the medical images by using the extracted surface information; and a synthesis image generator for generating a synthesis image in which the medical images have been registered, based on the mapping result.

According to one or more other embodiments, a robotic surgery system for performing a robotic surgery by a surgery robot by guiding images of a part to be operated includes: an endoscope apparatus for capturing medical images of a predetermined organ in a body to be examined; a non-endoscopic apparatus including at least one of an ultrasound apparatus, a computed tomography (CT) apparatus, a magnetic resonance imaging (MRI) apparatus, and a positron emission tomography (PET) apparatus for capturing medical images of the predetermined organ; a medical image processing apparatus for acquiring the medical images captured using the plurality of multi-modal medical image capturing apparatuses, extracting surface information of the predetermined organ, which is contained in each of the medical images, from each of the medical images, mapping the medical images by using the extracted surface information, and generating a synthesis image in which the medical images have been registered, based on the mapping result; a display apparatus for displaying the generated synthesis image; and the surgery robot for performing a robotic surgery according to a user input.

According to one or more other embodiments, a method of processing a medical image includes acquiring first image data of a predetermined organ in a living body using a first medical image capturing apparatus and second image data of the predetermined organ using a second medical image capturing apparatus that is different than the first,extracting first surface information of the predetermined organ from the first image data and second surface information of the predetermined organ from the second image data, matching a first position obtained from the first surface information to a second position obtained from the second surface information in accordance with relative position information of the first and second medical image capturing apparatuses, wherein the first position corresponds to the second position, generating a synthesis image from the first surface information and the second surface information based on the matching and outputting or displaying the synthesis image.

### BRIEF DESCRIPTION OF THE DRAWINGS

These and/or other aspects will become apparent and more readily appreciated from the following description of the embodiments, taken in conjunction with the accompanying drawings in which:
FIG. 1A is a block diagram of a robotic surgery system according to an embodiment of the present disclosure;
FIG. 1B is a block diagram of a robotic surgery system according to another embodiment of the present disclosure;
FIG. 2 is a conceptual diagram of relative positions of an endoscope apparatus and an ultrasound apparatus with respect to a bladder, according to an embodiment of the present disclosure;
FIG. 3 is a block diagram of a medical image processing apparatus according to an embodiment of the present disclosure;
FIG. 4 illustrates images to describe a process of extracting first surface information after generating a first surface model in a first extractor, according to an embodiment of the present disclosure;
FIG. 5 illustrates images to describe processes of extracting second surface information after generating a second surface model in a second extractor, according to an embodiment of the present disclosure;
FIG. 6 separately shows the arrangement of the endoscope apparatus and the ultrasound apparatus in the robotic surgery system of FIG. 1B;
FIG. 7 illustrates information included in a three-dimensional ultrasound image used to generate a synthesis image in a synthesis image generator, according to an embodiment of the present disclosure;
FIG. 8 illustrates a synthesis image according to an embodiment of the present disclosure;
FIG. 9 is a flowchart illustrating a method of processing a medical image, according to an embodiment of the present disclosure;
FIG. 10 is a detailed flowchart illustrating the medical image processing method of FIG. 9;
FIG. 11 is a flowchart illustrating a process of extracting first surface information, according to an embodiment of the present disclosure; and
FIG. 12 is a flowchart illustrating a process of extracting second surface information, according to an embodiment of the present disclosure.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

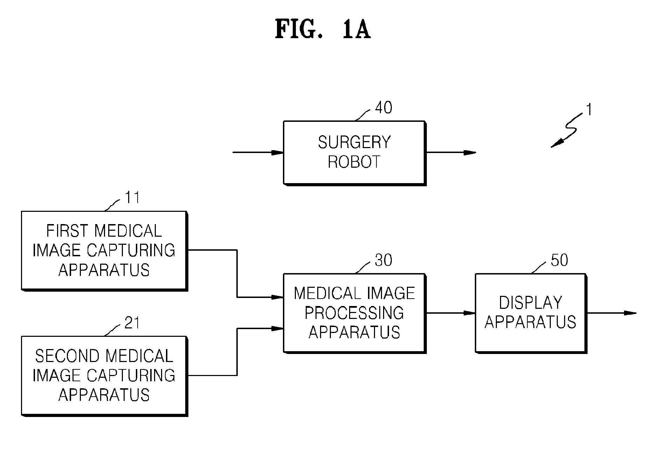

FIG. 1A is a block diagram of a robotic surgery system 1 according to an embodiment of the present disclosure. Referring to FIG. 1A, the robotic surgery system 1 may include, for example, a first medical image capturing apparatus 11, a second medical image capturing apparatus 21, a medical image processing apparatus 30, a surgery robot 40, and a display apparatus 50. In FIG. 1A and corresponding text, only hardware components associated with the current embodiment are described to keep aspects of the current embodiment from being obscured. However, it will be understood by those of ordinary skill in the art that the hardware components are described as examples only and that other general-use hardware components may be further included in the robotic surgery system 1.

Although the robotic surgery system 1 is illustrated and described as including first medical image capturing apparatus 11 and second medical image capturing apparatus 21 as medical image capturing apparatuses in FIG. 1A, the current embodiment is not limited thereto and may further include one or more other medical image capturing apparatuses.

There are various types of medical image capturing apparatuses, such as an endoscope apparatus, an ultrasound apparatus, a computed tomography (CT) apparatus, a magnetic resonance imaging (MRI) apparatus, and a positron emission tomography (PET) apparatus. Hereinafter, all described medical image capturing apparatuses, except for medical image capturing apparatuses for capturing endoscopic images, are called non-endoscopic apparatuses. That is, for example, the ultrasound apparatus, the CT apparatus, the MRI apparatus, and the PET apparatus are all described as non-endoscopic apparatuses.

Hereinafter, although for convenience of description and as an example the first medical image capturing apparatus 11 of the robotic surgery system 1 is described as an endoscope apparatus, such as a laparoscope apparatus, and the second medical image capturing apparatus 21 is described as an ultrasound apparatus, such as a trans-rectal ultrasound (TRUS) apparatus, the current embodiment is not limited thereto. That is, each of the first and second medical image capturing apparatuses 11 and 21 may be any one of the medical image capturing apparatuses including the endoscope apparatus, the ultrasound apparatus, the CT apparatus, the MRI apparatus, and the PET apparatus and the like.

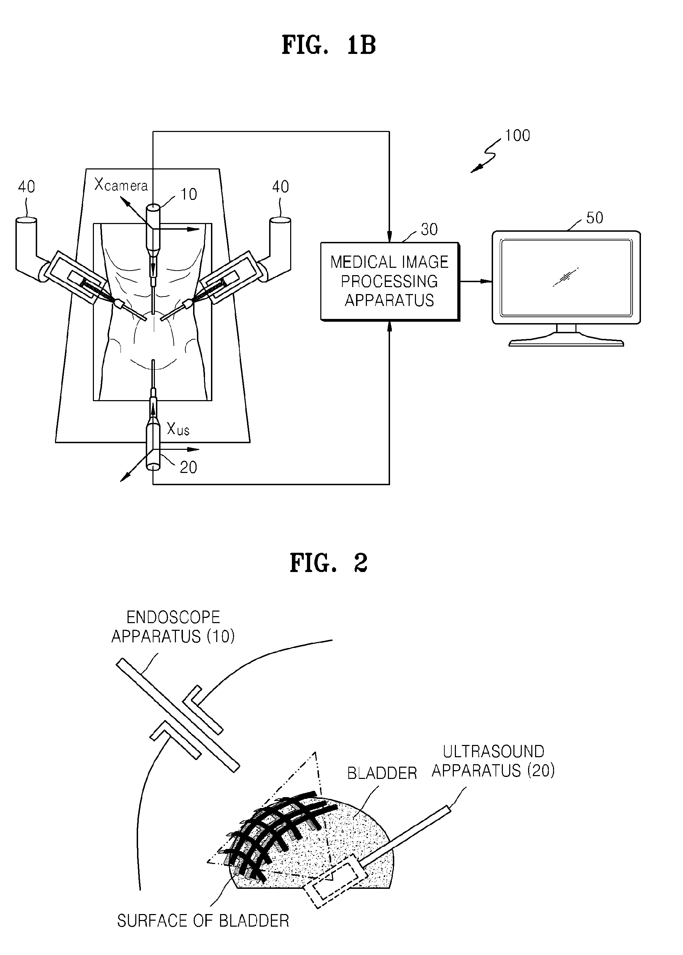

FIG. 1B is a block diagram of a robotic surgery system 100 according to another embodiment of the present disclosure. Referring to FIG. 1B, the robotic surgery system 100 may include, for example, an endoscope apparatus 10, an ultrasound apparatus 20, the medical image processing apparatus 30, the surgery robot 40, and the display apparatus 50, as described above.

In FIG. 1B as well as FIG. 1A, only hardware components associated with the current embodiment are described to keep aspects of the current embodiment from being obscured.

The robotic surgery system 100 is a system for operating on a patient by inserting arms of the surgery robot 40 through small holes made in the body of the patient and by controlling movements of the surgery robot 40. A surgeon who remains positioned outside of the body of the patient controls the movements of the surgery robot 40.

Today, the da Vinci® Surgical System ("da Vinci®") of Intuitive Surgical Inc. in the United States of America is typically used as the surgery robot 40. In more detail, da Vinci® is a robot including portions that are inserted directly into the body of a patient, the robot moving as if a surgeon were manually and directly operating on the patient. Although da Vinci® is illustrated as the surgery robot 40 in the current embodiment for convenience of description, the surgery robot 40 may correspond to another apparatus for performing a surgical operation (hereinafter, "operation" or "surgery") through movements of a robot inside the body of a patient.

When a surgeon desires to perform an operation using the surgery robot 40 of the robotic surgery system 100, the surgeon performs the operation by referring to medical images captured inside the body of a patient, which are displayed on the display apparatus 50. That is, when the robotic surgery system 100 is used, a surgeon operates after securing a view through images of nerve cells, blood vessels, and organs not always viewable with the naked eye by inserting a predetermined lens unit into the body of the patient.

In contrast with traditional, non-robotic abdominal operations, in robotic operations a surgeon typically perceives a part to be operated on by way of a screen displayed on the display apparatus 50 and without directly viewing the part to be operated on inside the body of a patient. Therefore, when using the robotic surgery system 100, a correct image of the part to be operated on is a requisite.

In particular, because a robotic surgery is typically performed to remove cancerous tissue such as in prostate cancer, rectal cancer, esophageal cancer, cervical, cancer, and bladder cancer operations, which may result in severe side effects and complications when surrounding nerve cells and blood vessels are damaged during an operation, it is necessary to display accurate and precise images of a part to be operated on using the display apparatus 50.

Until the present, a surgeon was typically required to view a CT, MRI, ultrasound, or PET image of a part to be operated on before surgery and to recall the part to be operated on from the stored diagnosis images during the surgery, or to view images captured prior to the surgery during the surgery. However, because these methods significantly depend on the experience of surgeons, it is difficult to perform the surgery correctly.

In addition, in some cases of existing robotic surgeries, the robotic surgeries are performed while viewing actual images inside the body of a patient, which are displayed by inserting an endoscope such as a laparoscope into the body. However, the images acquired by the laparoscope with respect to a part to be operated on are only images of the outer surfaces of organs. Thus, when a part or surface to be operated on is hidden by an organ or is inside an organ and not on an outer surface of the organ, it is difficult to acquire actual images of the part with the laparoscope. As a particular example, operations involving the prostate typically require access or viewing of a part or surface that is obscured by an organ or that is inside an organ and not on an outer surface of the organ.

In terms of a prostate operation, a prostate gland has a narrow part to be operated on and is connected to the urethra. In addition, when the prostate gland is removed, a neurovascular bundle around the prostate gland should be preserved because the neurovascular bundle is needed to maintain urinary function and sexual function. However, because the laparoscope provides only images of external surface tissues, it is difficult to accurately and precisely perceive a position and shape of the prostate gland when using just the laparoscope and thereby avoid damage to the neurovascular bundle.

Although a trans-rectal ultrasound (TRUS) apparatus has been used to improve these existing methods, there is a limitation in that ultrasound images of the part to be operated on are provided instead of actual images thereof.

In addition, by detecting a position and orientation of a TRUS apparatus in real-time using a marker in an optical or magnetic field scheme, three-dimensional (3D) ultrasound images have been acquired and used in an operation. However, when the magnetic field scheme is used, position measurement of the marker may be incorrect due to magnetic field interference between the marker and a metallic material, such as a surgery robot. When the optical scheme is used, the movements of a surgery robot are limited because a range of the marker overlaps a movement range of the surgery robot when a position of the marker is measured.

A technique of rotating a TRUS apparatus by a tandem robot and acquiring 3D prostate images from the TRUS apparatus has been also used. However, because this method uses the additional tandem robot, the movements of a surgery robot may be limited by interference with the tandem robot. In addition, because of compensation needed for positions of the surgery robot and the tandem robot, ultrasound images may not be fully used in an actual operation.

That is, as described above, when robotic surgeries are performed, and in particular, when robotic surgeries in which it is desirable to prevent surrounding nerve cells and blood vessels from being damaged are performed, such as in prostate robotic surgeries, correct images of a part to be operated on have rarely been acquired. Therefore, the safety of the patient cannot be guaranteed.

However, in the robotic surgery system 100, according to one or more current embodiments, a synthesis image in which multi-modal medical images captured by a plurality of multi-modal medical image capturing apparatuses are registered in real time. That is, the captured multi-modal medical images of the synthesis image are registered so that identical positions of the organ correspond in each of the multi-modal medical images. Thus, correct images of a part to be operated on may be provided, even when the part to be operated on is hidden by an organ or is located inside an organ, thereby guaranteeing the performance of a robotic surgery or the safety of a patient.

Although it is described in the current embodiments that the medical images are provided for a surgeon performing a robotic surgery with the robotic surgery system 100, that is, medical image guidance is described, a synthesis image generated by the medical image processing apparatus 30 is not limited to that provided in the robotic surgery system 100. That is, medical images provided in the current embodiment may be provided in other systems for simply examining or diagnosing a patient as well as in robotic surgeries.

Hereinafter, a process of processing a medical image in the robotic surgery system 100 according to one or more embodiments is described in detail.

As an example, a prostate gland is illustrated as a part to be operated on. When the prostate gland is operated on, a predetermined organ, e.g., a bladder or a rectum, located around the prostate gland, is used to describe the medical image processing according to the current embodiment. That is, the predetermined organ may be an organ corresponding to the part to be operated on by the surgery robot 40 or another organ around the part to be operated on.

Furthermore, it will be understood by those of ordinary skill in the art that the part to be operated on may be another part of a patient or the medical image processing may be performed using another organ.

Referring back to FIG. 1B, the endoscope apparatus 10 in the robotic surgery system 100 acquires an endoscopic image of the organ, e.g., the bladder, of the patient. Thus, the endoscopic image includes images of the bladder of the patient and the surroundings of the bladder. Although the endoscope apparatus 10 in the current embodiment may correspond to a laparoscope apparatus, the endoscope apparatus 10 is not limited thereto.

The ultrasound apparatus 20 acquires an ultrasound image of the bladder of the patient and the surroundings of the bladder such as a real-time ultrasound image obtained during surgery. Thus, the ultrasound image includes images of the bladder of the patient and the surroundings inside and outside the bladder. That is, unlike the endoscopic image, the ultrasound image may include information regarding tissues inside the bladder. Although the ultrasound apparatus 20 in the current embodiment may correspond to a TRUS apparatus, the ultrasound apparatus 20 is not limited thereto.

In FIG. 1B, the endoscope apparatus 10 and the ultrasound apparatus 20 capture medical images at different positions. That is, in the robotic surgery system 100, medical images are captured by individually controlling movements and positions of the endoscope apparatus 10 and the ultrasound apparatus 20. At this time, the robotic surgery system 100 continuously stores captured positions, e.g., virtual coordinates on a robotic surgery table, of the endoscope apparatus 10 and the ultrasound apparatus 20 in a storage unit (not shown) of the robotic surgery system 100.

FIG. 2 is a conceptual diagram illustrating relative positions of the endoscope apparatus 10 and the ultrasound apparatus 20 with respect to the bladder, according to an embodiment of the present disclosure. Referring to FIG. 2, when the endoscope apparatus 10 is a laparoscope apparatus, the endoscope apparatus 10 acquires the endoscopic image from a position located relatively higher than or above the bladder. In addition, when the ultrasound apparatus 20 is a TRUS apparatus, the ultrasound apparatus 20 acquires the ultrasound image from a position located relatively lower than or below the bladder. However, the positions of the endoscope apparatus 10 and the ultrasound apparatus 20 are only illustrative and may be changed according to a robotic surgery environment.

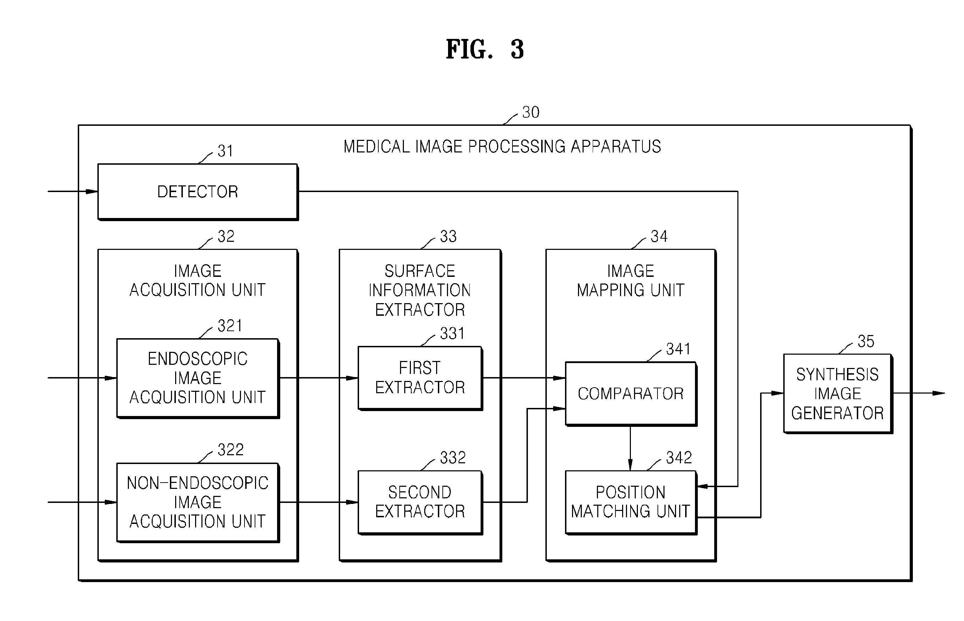

Referring back to FIG. 1B, the medical image processing apparatus 30 may generate a synthesis image by registering and synthesizing the endoscopic image and the ultrasound image respectively acquired from the endoscope apparatus 10 and the ultrasound apparatus 20. An operation and function of the medical image processing apparatus 30 is described in detail with reference to FIG. 3.

FIG. 3 is a block diagram of the medical image processing apparatus 30 according to an embodiment of the present disclosure. Referring to FIG. 3, the medical image processing apparatus 30 may include, for example, a detector 31, an image acquisition unit 32, a surface information extractor 33, an image mapping unit 34, and a synthesis image generator 35. The image acquisition unit 32 may include an endoscopic image acquisition unit 321 and a non-endoscopic image acquisition unit 322, the surface information extractor 33 may include a first extractor 331 and a second extractor 332, and the image mapping unit 34 may include a comparator 341 and a position matching unit 342.

The medical image processing apparatus 30 may correspond to a processor, which may be implemented by an array of logic gates or by a combination of a general-use microprocessor and a memory storing programs executable by the general-use microprocessor. In addition, it will be understood by those of ordinary skill in the art that the medical image processing apparatus 30 may be implemented by another type of hardware.

When a synthesis image is to be generated, the detector 31 may detect current positions of medical image capturing apparatuses that are stored in the storage unit (not shown) of the robotic surgery system 100 described above.

The image acquisition unit 32 may acquire medical images, for example, an endoscopic image and a non-endoscopic image, of an organ, which are captured using a plurality of multi-modal medical image capturing apparatuses.

The surface information extractor 33 may extract surface information of the organ, which is included in each of the medical images, from each of the medical images. In particular, the surface information extractor 33 may extract information indicating at least one of a position and a shape of the surface of the organ as the surface information from each of the medical images.

The image mapping unit 34 may map the medical images using the extracted surface information. In particular, the image mapping unit 34 may map the medical images by matching the positions of the medical image capturing apparatuses with each other using the extracted surface information.

Hereinafter, a process of processing an endoscopic image is described in more detail, and then a process of processing a non-endoscopic image, such as an ultrasound image, a CT image, and a magnetic resonance (MRI) image, is described in more detail.

The endoscopic image acquisition unit 321 acquires an endoscopic image captured using the endoscope apparatus such as shown at item 10 of FIG. 1B).

The first extractor 331 may extract first surface information indicating at least one of a position and a shape of the surface of the organ from the endoscopic image captured by the endoscope apparatus (10 of FIG. 1B). That is, in the current embodiment, the first extractor 331 extracts first surface information regarding the bladder represented as the endoscopic image.

In detail, the first extractor 331 may generate a disparity space image by acquiring distance information between external tissues of the bladder and its surroundings and the endoscope apparatus 10. According to an embodiment of the present disclosure, the first extractor 331 may generate the disparity space image by using the endoscope apparatus 10 including two stereoscopic cameras. According to another embodiment of the present disclosure, the first extractor 331 may generate the disparity space image by using the endoscope apparatus 10 further including a projector for radiating at least one of a structured-light and a patterned-light. In this case, the endoscopic image acquisition unit 321 may also acquire information regarding structured-light or patterned-light reflected from the external tissues of the bladder and its surroundings. That is, the first extractor 331 may calculate a distance from the endoscope apparatus 10 to the external tissues of the bladder and its surroundings by using the information regarding structured-light or patterned-light. Thus, the first extractor 331 may generate a distance image, such as a disparity space image, based on the calculated distance.

Thereafter, the first extractor 331 may generate a 3D first surface model corresponding to the endoscopic image using the acquired distance information such as the calculated distance or the generated distance image.

Finally, the first extractor 331 may extract the first surface information indicating at least one of a position and a shape of the surface of the bladder from the first surface model.

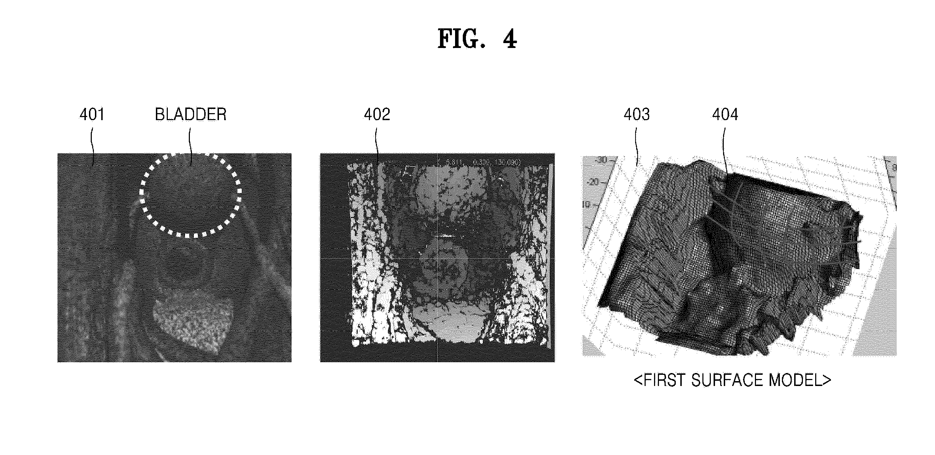

FIG. 4 illustrates images 401, 402, and 403 to describe a process of extracting first surface information 404 after generating a first surface model in the first extractor (331 of FIG. 3), according to an embodiment of the present disclosure.

The image 401 of FIG. 4 is an example of an endoscopic image acquired by the endoscope apparatus 10, i.e., an actual image obtained by radiating structured-light or patterned-light onto the bladder and its surroundings.

The image 402 of FIG. 4 is a disparity space image corresponding to a distance image generated by using at least one of the structured-light and the patterned-light. However, as described above, the first extractor (331 of FIG. 3) may extract a disparity space image without using the structured-light or the patterned-light.

The image 403 of FIG. 4 shows the first surface model generated by the first extractor (331 of FIG. 3) through the above-described process. The first extractor (331 of FIG. 3) extracts the first surface information 404 regarding the shape and position of the surface of the bladder from the first surface model of the image 403.

Referring back to FIG. 3, the non-endoscopic image acquisition unit 322 may acquire a non-endoscopic image captured using a non-endoscopic apparatus, such as the ultrasound apparatus (20 of FIG. 1B).

The second extractor 332 may extract second surface information indicating at least one of a position and a shape of the surface of the organ from the non-endoscopic image captured by the non-endoscopic apparatus. That is, in the current embodiment, the second extractor 332 may extract second surface information regarding the bladder represented as the non-endoscopic image.

In detail, first, the second extractor 332 acquires information regarding a boundary indicating the surface of the bladder from the non-endoscopic image captured by the non-endoscopic apparatus. At this time, the information regarding a boundary is acquired by applying at least one of line detection and edge detection to the non-endoscopic image.

When the non-endoscopic image is an ultrasound image, the characteristic of ultrasound to have high echogenicity with respect to surface tissues of an organ is utilized. That is, the second extractor 332 acquires the information regarding a boundary using the fact that surface tissues of an organ are shown as relatively bright lines in an ultrasound image.

When the non-endoscopic image is an MRI image, the second extractor 332 acquires the information regarding a boundary by using line detection or edge detection, based on the fact that an image contrast occurs in the MRI image due to a difference between molecular structure ratios of tissues.

Likewise, when the non-endoscopic image is a CT image, the second extractor 332 acquires the information regarding a boundary by using line detection or edge detection, based on the fact that an image contrast occurs in the CT image due to a density difference between tissues.

Thereafter, the second extractor 332 may generate a 3D second surface model corresponding to the surface of the organ (bladder) by using the acquired boundary information. At this time, the second extractor 332 may generate a 3D second surface model by three-dimensionally rendering boundaries based on the acquired boundary information.

Finally, the second extractor 332 may extract the second surface information indicating at least one of a position and a shape of the surface of the bladder from the second surface model.

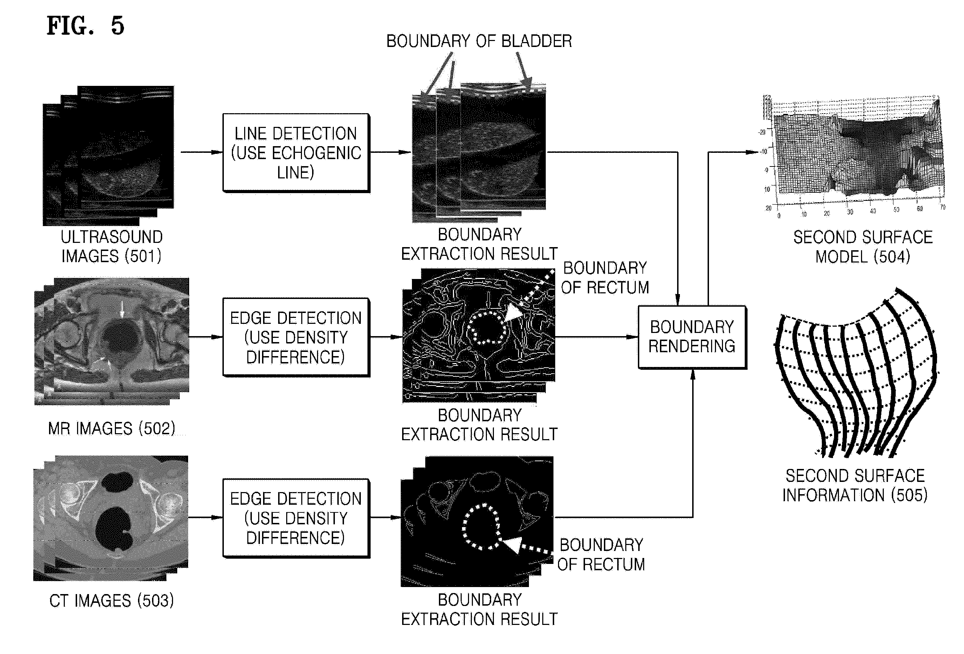

FIG. 5 illustrates images to describe processes of extracting second surface information 505 after generating a second surface model 504 in the second extractor (332 of FIG. 3), according to an embodiment of the present disclosure.

Referring to FIG. 5, a process of extracting the second surface information 505 from ultrasound images 501, a process of extracting the second surface information 505 from MRI images 502, and a process of extracting the second surface information 505 from CT images 503 are illustrated. According to which type of medical image capturing apparatus is used in the environment of the robotic surgery system (100 of FIG. 1B), the second extractor 332 may extract the second surface information 505 based on a corresponding process of the above-described processes.

When the ultrasound images 501 are used, the second extractor 332 extracts a boundary corresponding to the surface of the bladder from each of the ultrasound images 501 by using the ultrasound image characteristics described above and generates the second surface model 504 by three-dimensionally rendering each of the boundaries.

When the MRI images 502 are used, the second extractor 332 extracts a boundary corresponding to the surface of the rectum from each of the MRI images 502 by using the MRI image characteristics described above and generates the second surface model 504 by three-dimensionally rendering each of the boundaries.

When the CT images 503 are used, the second extractor 332 extracts a boundary corresponding to the surface of the rectum from each of the CT images 503 by using the CT image characteristics described above and generates the second surface model 504 by three-dimensionally rendering each of the boundaries.

The second extractor 332 extracts the second surface information 505 indicating at least one of the shape and position of the organ based on the boundary information represented from the second surface model 504.

That is, according to the type of medical image capturing apparatus that is used in the environment of the robotic surgery system (100 of FIG. 1B), the second extractor (332 of FIG. 3) may extract the second surface information 505 based on a corresponding one of the processes for the ultrasound images 501, the MRI images 502, and the CT images 503.

Referring back to FIG. 3, the image mapping unit 34 may map the medical images using the extracted surface information.

In detail, the image mapping unit 34 may map the medical images by matching the positions of the medical image capturing apparatuses with each other using the extracted first surface information and the extracted second surface information. As shown in FIG. 1B, when the endoscope apparatus 10 and the ultrasound apparatus 20 are used, the endoscopic image and the ultrasound image are mapped by matching the positions of the organ obtained from the endoscope apparatus 10 and the ultrasound apparatus 20using the first and second surface information.

A mapping process is described in more detail. The image mapping unit 34 may include, for example, the comparator 341 and the position matching unit 342 as described above.

The comparator 341 may compare the first surface information with the second surface information because the first surface information and the second surface information each provide information regarding the surface of the same part of the organ (bladder). Thus, the comparator 341 compares the first surface information with the second surface information with respect to the surface of the same part of the organ. At this time, the comparator 341 may use a well-known algorithm, such as the Iterative Closest Point (ICP) algorithm, to perform the comparison or may use other algorithms.

The position matching unit 342 may match the positions of the medical image capturing apparatuses, which are detected by the detector 31, with each other based on the comparison result.

As a result, the image mapping unit 34 maps the medical images based on the matching result.

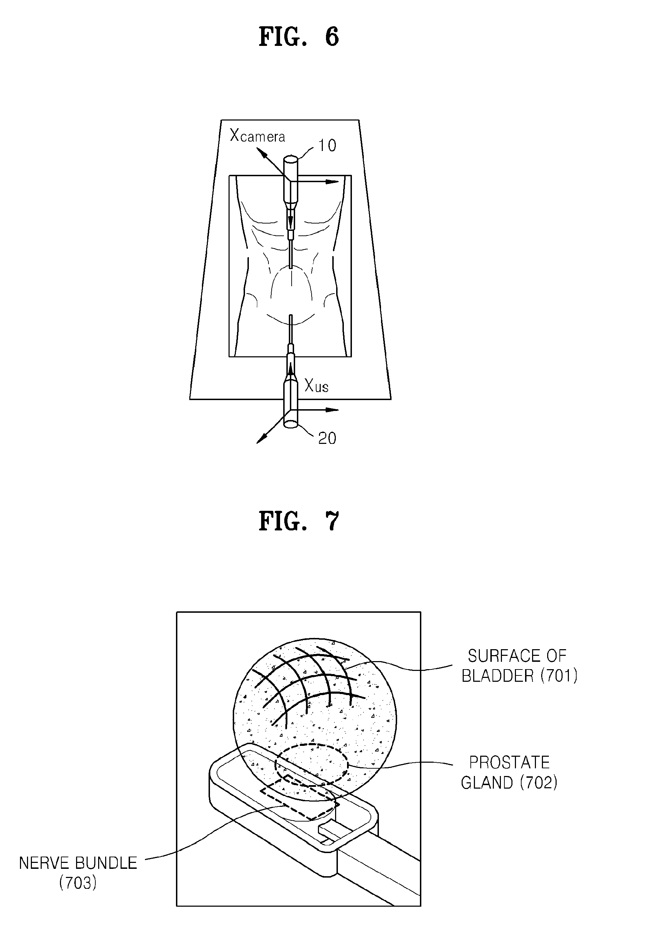

FIG. 6 separately shows an example arrangement of the endoscope apparatus 10 and the ultrasound apparatus 20 in the robotic surgery system 100 of FIG. 1B. Referring to FIG. 6, the endoscope apparatus 10 corresponds to a laparoscope apparatus, and the ultrasound apparatus 20 corresponds to a TRUS apparatus.

In the robotic surgery system 100, a virtual position of the endoscope apparatus 10 conforms to an X_{camera} coordinate system, and a virtual position of the ultrasound apparatus 20 conforms to an X_{US} coordinate system. That is, because a position of the endoscope apparatus 10 and a position of the ultrasound apparatus 20 are represented using different coordinate systems, the positions of the endoscope apparatus 10 and the ultrasound apparatus 20 are independent from each other.

However, the position of the endoscope apparatus 10 and the position of the ultrasound apparatus 20 may be matched with each other based on the same criteria. To do this, the image mapping unit 34 may use the first surface information and the second surface information as the criteria according to the current embodiment. In more detail, the first surface information and the second surface information each include information regarding the surface of the same part of the organ (bladder). Thus, the X_{camera} coordinate system and the X_{US} coordinate system may be matched with each other based on the first surface information extracted from the endoscopic image and the second surface information extracted from the ultrasound image. As a result, the position of the endoscope apparatus 10 and the position of the ultrasound apparatus 20 may also be matched with each other.

Referring back to FIG. 3, the synthesis image generator 35 may generate a synthesis image in which the medical images are registered, based on the mapping result. That is, the synthesis image generator 35 may generate the synthesis image in which the medical images are registered so that identical positions of the organ correspond in each of the multi-modal medical images. The generated synthesis image may be a 3D medical image related to the organ and its surroundings. In more detail, the generated synthesis image is an image obtained by three-dimensionally representing both an image of external tissues of the organ and its surroundings included in the image captured by the endoscope apparatus 10 and an image of tissues inside and outside the organ and its surroundings included in the image captured by the non-endoscopic apparatus 20. The generated synthesis image may correspond to a kind of augmented reality image derived from the synthesis of each of the multi-modal medical images.

The synthesis image generated by the synthesis image generator 35 is eventually generated by registering the endoscopic image and the non-endoscopic image so that the position of the organ is the same in the endoscopic image and the non-endoscopic image.

An endoscopic image itself corresponds to an actual 3D image of an organ and its surroundings. However, it is difficult to perceive information regarding shapes and positions of tissues inside and outside the organ from the endoscopic image. For example, it is difficult to perceive information regarding shapes and positions of tissue internal to the organ or located behind other tissue.

In general, a non-endoscopic image may correspond to a set of cross-sectional images of an organ. However, the non-endoscopic image, such as ultrasound images, CT images, or MRI images, includes a kind of fluoroscopic information with respect to tissues inside and outside the organ and its surroundings. Thus, the acquired non-endoscopic image includes information regarding shapes and positions of tissues inside and outside the organ and tissue hidden behind other tissue. Accordingly, when the endoscopic image and the non-endoscopic image are synthesized, information regarding tissues inside and outside an actual organ and its surroundings may be correctly perceived by a surgeon so that the surgeon performs a relatively accurate and precise operation.

The non-endoscopic image, such as ultrasound images, CT images, or MRI images, may be a 2D image or a 3D image according to the type of the medical image capturing apparatuses (11 and 21 of FIG. 1A) that is used. If the acquired non-endoscopic image corresponds to a plurality of 2D non-endoscopic images as shown in FIG. 5, the synthesis image generator 35 may generate a 3D non-endoscopic image from the 2D non-endoscopic images by using any of a variety of well-known methods, such as volume rendering, to generate the synthesis image.

FIG. 7 illustrates information that may be included in a 3D ultrasound image used to generate a synthesis image in the synthesis image generator 35, according to an embodiment of the present disclosure. FIG. 7 illustrates an example in which a TRUS apparatus is used. Referring to FIG. 7, an ultrasound image includes a kind of fluoroscopic information with respect to shapes and positions of tissues inside and outside an organ, e.g., the bladder, as described above. Thus, the 3D ultrasound image three-dimensionally shows a shape and position of the bladder's outer surface 701, a position of a prostate gland 702, and a position of a nerve bundle 703 around the bladder. The shape and position of the bladder's outer surface 701 is information included in the second surface information. Although FIG. 7 is not actually a 3D ultrasound image, it will be understood by those of ordinary skill in the art that the 3D ultrasound image typically includes these types of information (information corresponding to reference numerals 701, 702, and 703).

Referring back to FIG. 3, the synthesis image generator 35 may generate a synthesis image by registering the endoscopic image and the non-endoscopic image.

Referring back to FIGS. 1A and 1B, the display apparatus 50 may be used to display the synthesis image generated by the synthesis image generator 35. The robotic surgery system 1 or 100 performs image guidance using the display apparatus 50 by providing the synthesis image to a surgeon performing a robotic surgery. The display apparatus 50 includes a device for displaying visual information such as a general-use monitor, a Liquid Crystal Display (LCD) display, a Light Emitting Diode (LED) display, or a scale display device, to provide information to a user.

Referring back to FIG. 3, different images of the same organ, which are acquired by different apparatuses at different positions, may be continuously mapped to each other in real time if the position matching unit 342 matches the position of the endoscope apparatus (10 of FIG. 1B) and the position of the ultrasound apparatus (20 of FIG. 1B) in real time using the first surface information and the second surface information. Thus, the synthesis image generator 35 may continuously generate synthesis images by continuously registering endoscopic images and non-endoscopic images, so that the display apparatus 50 displays in real time the synthesis images regardless of movements of the endoscope apparatus (10 of FIG. 1B) and the ultrasound apparatus (20 of FIG. 1B) .

According to an embodiment of the present disclosure, although the display apparatus 50 displays the generated synthesis image as it is, the display apparatus 50 may be controlled to display only a partial area of interest among image information included in the synthesis image according to a use environment of the robotic surgery system 1 or 100. That is, when an endoscopic image and a non-endoscopic image are synthesized, the display apparatus 50 may, in an embodiment, be controlled to display only a position of a prostate gland 801 and positions of nerve bundles 802 that are partial areas of interest, which are included in the non-endoscopic image. Furthermore, if information regarding the positions of the prostate gland 801 and the nerve bundles 802 is pre-processed, the display apparatus 50 may display the synthesis image together with information that a predetermined part corresponds to the prostate gland 801 and the nerve bundles 802.

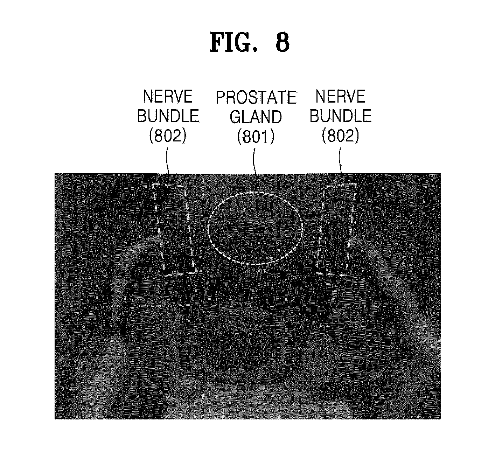

FIG. 8 illustrates a synthesis image according to an embodiment of the present disclosure. Referring to FIG. 8, the synthesis image is obtained by synthesizing an endoscopic image of a bladder & its surroundings and an ultrasound image having information regarding positions of tissues, such as the prostate gland 801 and the nerve bundles 802, inside and outside the bladder.

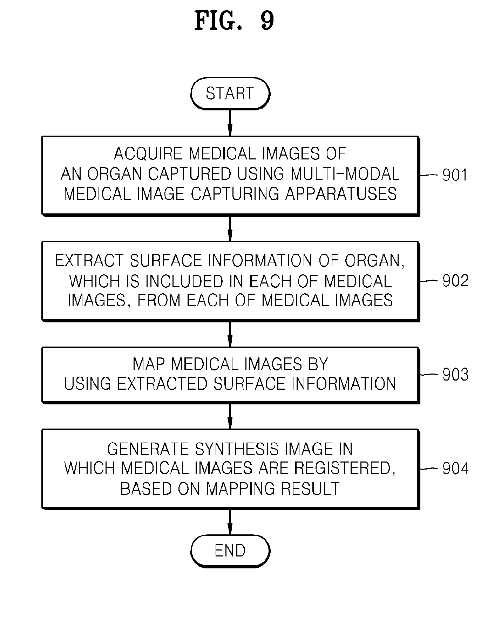

FIG. 9 is a flowchart illustrating a method of processing a medical image, according to an embodiment of the present disclosure. Referring to FIG. 9, the medical image processing method according to the current embodiment may include sequential operations processed by the medical image processing apparatus 30 of the robotic surgery system 1 or 100 that are shown in FIGS. 1A, 1B, and 3. Thus, although omitted below, the content described with reference to FIGS. 1A, 1B, and 3 also applies to the medical image processing method according to the current embodiment.

In operation 901, the image acquisition unit 32 acquires medical images of an organ captured using a plurality of multi-modal medical image capturing apparatuses.

In operation 902, the surface information extractor 33 extracts surface information of the organ, which is included in each of the medical images, from each of the medical images.

In operation 903, the image mapping unit 34 maps the medical images using the extracted surface information.

In operation 904, the synthesis image generator 35 generates a synthesis image in which the medical images are registered, based on the mapping result.

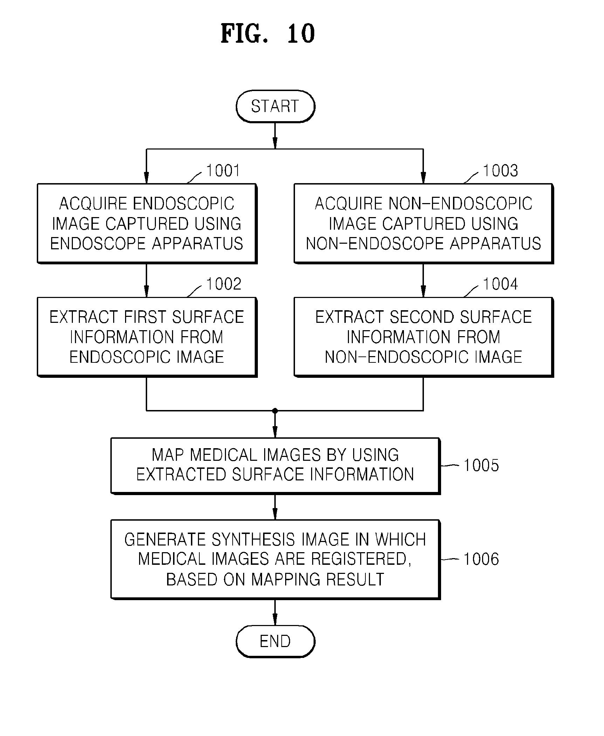

FIG. 10 is a detailed flowchart illustrating the medical image processing method of FIG. 9, according to an embodiment of the present disclosure. Likewise, although omitted below, the content described with reference to FIGS. 1A, 1B, and 3 also applies to the medical image processing method according to the current embodiment.

In operation 1001, an endoscopic image captured using the endoscope apparatus 10 is acquired, for example, by the endoscopic image acquisition unit 321.

In operation 1002, first surface information indicating at least one of a position and a shape of the surface of the organ is extracted from the endoscopic image captured by the endoscope apparatus 10, for example by the first extractor 331.

In operation 1003, a non-endoscopic image captured using a non-endoscopic apparatus, such as the ultrasound apparatus 20 is acquired, for example by the non-endoscopic image acquisition unit 322.

In operation 1004, second surface information indicating at least one of a position and a shape of the surface of the organ from the non-endoscopic image captured by the non-endoscopic apparatus is extracted, for example by the second extractor 332.

Operations 1001 and 1003 may be simultaneously performed in parallel, or either of operations 1001 and 1003 may be performed first. That is, operations 1001 and 1002 may be independently performed from operations 1003 and 1004 without affecting each other.

In operation 1005, the medical images are mapped using the first surface information and the second surface information, for example, by the image mapping unit 34.

In operation 1006, a synthesis image in which the medical images are registered is generated, based on the mapping result, for example by the synthesis image generator 35.

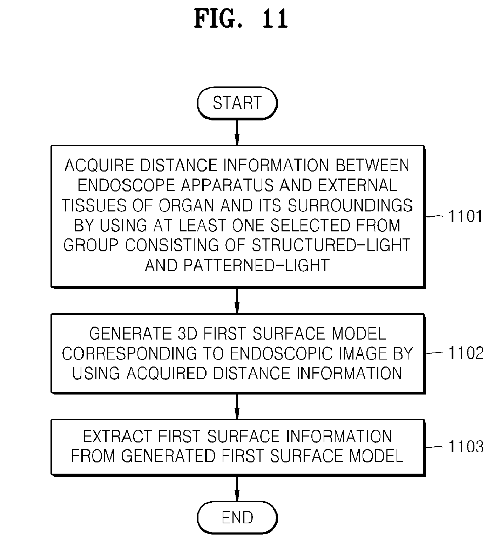

FIG. 11 is a flowchart illustrating a process of extracting the first surface information, according to an embodiment of the present disclosure.

Referring to FIG. 11, in operation 1101, distance information between the external tissues of the organ and its surroundings and the endoscope apparatus 10 is acquired, for example by the first extractor 331.

In operation 1102, a 3D first surface model corresponding to the endoscopic image is generated using the acquired distance information, for example by the first extractor 331.

In operation 1103, the first extractor 331 may then extract the first surface information from the generated first surface model.

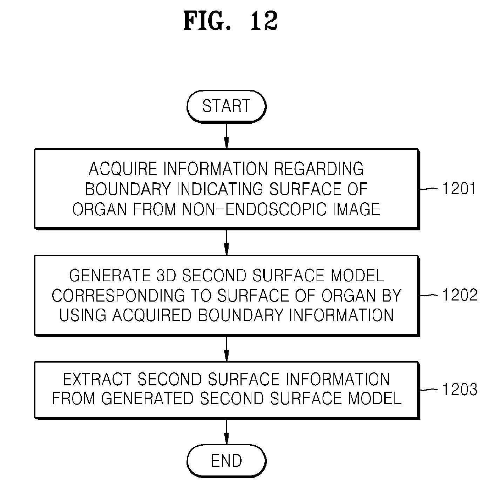

FIG. 12 is a flowchart illustrating a process of extracting the second surface information, according to an embodiment of the present disclosure.

Referring to FIG. 12, in operation 1201, information regarding a boundary indicating the surface of the organ from the non-endoscopic image captured by the non-endoscopic apparatus 20 is acquired, for example by the second extractor 332.

In operation 1202, the second extractor 332 may generate a 3D second surface model corresponding to the surface of the organ by using the acquired boundary information.

In operation 1203, the second extractor 332 may extract the second surface information from the generated second surface model.

As described above, according to the one or more of the above embodiments of the present disclosure, complications and inconvenience caused by the use of an artificial marker in image registration may be reduced by registering medical images in real time based on information included in the medical images instead of using the artificial marker. In particular, a decrease in image registration accuracy due to interference between a metallic marker and a surgery robot during a robotic surgery may be reduced.

In addition, by generating in real time a synthesis image in which an endoscopic image and a non-endoscopic image are registered, a correct diagnosis image of a patient may be provided to a surgeon, or correct image guidance in a robotic surgery system may be provided. Accordingly, by providing correct medical images of a part to be operated on in a robotic surgery, the part to be operated on and a part to be preserved may be correctly perceived, thereby improving operation performance. Furthermore, when a robotic surgery is automated in the future, information for correctly controlling a robot may be provided.

The embodiments of the present disclosure may be written as computer programs or program instructions and may be implemented in general-use digital computers that execute the programs using a non-transitory computer-readable recording medium. In addition, data structures used in the embodiments of the present disclosure may be recorded in the computer-readable recording medium in various ways.

The media may also include, alone or in combination with the program instructions, data files, data structures, and the like. Examples of non-transitory computer-readable media include magnetic media such as hard disks, floppy disks, and magnetic tape; optical media such as CD ROM discs and DVDs; magneto-optical media such as optical discs; and hardware devices that are specially configured to store and perform program instructions, such as read-only memory (ROM), random access memory (RAM), flash memory, and the like.

Examples of program instructions include both machine code, such as produced by a compiler, and files containing higher level code that may be executed by the computer using an interpreter. The described hardware devices may be configured to act as one or more software modules in order to perform the operations of the above-described embodiments, or vice versa. Any one or more of the software modules described herein may be executed by a dedicated processor unique to that unit or by a processor common to one or more of the modules. The described methods may be executed on a general purpose computer or processor or may be executed on a particular machine such as the apparatus described herein.

While the present disclosure has been particularly shown and described with reference to exemplary embodiments thereof, it will be understood by those of ordinary skill in the art that various changes in form and details may be made therein without departing from the spirit and scope of the present disclosure as defined by the following claims. The exemplary embodiments should be considered in descriptive sense only and not for purposes of limitation. Therefore, the scope of the present disclosure is defined not by the detailed description of the present disclosure but by the appended claims, and all differences within the scope will be construed as being included in the present disclosure.

Although a few embodiments have been shown and described, it would be appreciated by those skilled in the art that changes may be made in these embodiments without departing from the principles and spirit of the disclosure, the scope of which is defined in the claims and their equivalents.

## Claims

1. A method of processing a medical image, the method comprising:
acquiring medical images captured using a plurality of different multi-modal medical image capturing apparatuses with respect to a predetermined organ;
extracting surface information of the predetermined organ, which is included in each of the medical images, from each of the medical images;
mapping each of the medical images using the extracted surface information; and
generating a synthesis image in which the medical images have been registered, based on the mapping result.

2. The method of claim 1, wherein the extracting of the surface information comprises extracting information indicating at least one of a position and a shape of the surface of the predetermined organ from each of the medical images.

3. The method of claim 1 or 2, wherein the mapping of each of the medical images comprises mapping each of the medical images by matching positions of the medical image capturing apparatuses with each other using the extracted surface information.

4. The method of any of the preceding claims 1-3, further comprising detecting positions of the medical image capturing apparatuses,
wherein the mapping of the medical images comprises:
comparing the extracted surface information with each other; and
matching the positions of the medical image capturing apparatuses with each other based on the comparison result, and
the mapping of the medical images comprises mapping the medical image capturing apparatuses based on the matching result.

5. The method of claim 4, wherein the comparing of the extracted surface information comprises comparing the extracted surface information with each other with respect to a surface of a same portion of the predetermined organ.

6. The method of any of the preceding claims 1-5,
wherein the plurality of multi-modal medical image capturing apparatuses comprises an endoscope apparatus and a non-endoscopic apparatus comprising at least one of an ultrasound apparatus, a computed tomography (CT) apparatus, a magnetic resonance imaging (MRI) apparatus, and a positron emission tomography (PET) apparatus.

7. The method of any of the preceding claims 1-6,
wherein the generated synthesis image is an image in which an image of tissues outside the predetermined organ and surroundings of the tissues outside the predetermined organ included in an image captured by the endoscope apparatus and an image of tissues inside and outside the predetermined organ and surroundings of the tissues inside and outside the predetermined organ included in an image captured by the non-endoscopic apparatus are three-dimensionally represented at the same time.

8. The method of any of the preceding claims 1-7,
wherein the extracting of the surface information comprises:
extracting first surface information indicating at least one of a position and a shape of the surface of the predetermined organ from an endoscopic image captured by the endoscope apparatus; and
extracting second surface information indicating at least one of a position and a shape of the surface of the predetermined organ from a non-endoscopic image captured by the non-endoscopic apparatus.

9. The method of claim 8, wherein the extracting of the first surface information comprises:
acquiring distance information between external tissues of the predetermined organ and its surroundings and the endoscope apparatus;
generating a three-dimensional first surface model corresponding to the endoscopic image using the acquired distance information; and
extracting the first surface information from the generated three-dimensional first surface model.

10. The method of claim 8, wherein the extracting of the second surface information comprises:
acquiring information regarding a boundary indicating the surface of the predetermined organ from the image captured by the non-endoscopic apparatus;
generating a three-dimensional second surface model corresponding to the surface of the predetermined organ using the acquired boundary information; and
extracting the second surface information from the generated three-dimensional second surface model.

11. A computer-readable recording medium storing a computer-readable program for executing the method of any one of claims 1 to 10.

12. An apparatus for processing a medical image, the apparatus comprising:
an image acquisition unit for acquiring medical images captured using a plurality of different multi-modal medical image capturing apparatuses with respect to a predetermined organ;
a surface information extractor for extracting surface information of the predetermined organ, which is included in each of the medical images, from each of the medical images;
an image mapping unit for mapping each of the medical images using the extracted surface information; and
a synthesis image generator for generating a synthesis image in which the medical images have been registered, based on the mapping result.

13. The apparatus of claim 12, wherein the surface information extractor extracts information indicating at least one of a position and a shape of the surface of the predetermined organ as the surface information from each of the medical images.

14. The apparatus of claim 12 or 13, wherein the image mapping unit maps each of the medical images by matching positions of the medical image capturing apparatuses with each other using the extracted surface information.

15. The apparatus of claim 12, 13 or 14, further comprising a detector for detecting positions of the medical image capturing apparatuses,
wherein the image mapping unit comprises:
a comparator for comparing the extracted surface information with each other; and
a position matching unit for matching the positions of the medical image capturing apparatuses with each other based on the comparison result.
